# EUROPEAN PATENT APPLICATION

(11) **EP 4 640 209 A1**
(43) Date of publication of application: **29.10.2025**
(21) Application number: 23907412.3
(22) Date of filing: 10.11.2023
(51) Int. Cl.: A61K 8/9789, A61K 8/49, A61Q 19/00

(54) **COSMETIC COMPOSITION CONTAINING HOUTTUYNIA CORDATA FERMENTATION EXTRACT**

(30) Priority: 21.12.2022 KR 20220179961
(71) Applicant: Daebong LS Co., Ltd., Incheon 21697 (KR); UCL Co. Ltd., Jeju-si, Jeju-do 63038 (KR)
(72) Inventor: KIM, Keon-Woo, Incheon 21910 (KR); KIM, Jeong-Mi, Jeju-si, Jeju-do 63137 (KR); MOON, Mi-So, Jeju-si, Jeju-do 63072 (KR); MOON, Ji-Young, Jeju-si, Jeju-do 63098 (KR); PARK, Jin-Oh, Seoul 06276 (KR); YEOM, Hyun-Sook, Jeju-si, Jeju-do 63293 (KR); OH, Tae-Heon, Jeju-si, Jeju-do 63098 (KR); HWANG, Jin, Jeju-si, Jeju-do 63236 (KR); OH, Dae-Ju, Jeju-si, Jeju-do 63278 (KR); LEE, Yoon-Ji, Seogwipo-si, Jeju-do 63608 (KR)
(74) Representative: Plasseraud IP
(86) International application number: PCT/KR2023/018054
(87) International publication number: WO 2024/136126

(57) **Abstract**

The present disclosure relates to a cosmetic composition containing a *Houttuynia cordata* fermentation extract fermented with lactic acid bacteria. The *Houttuynia cordata* fermentation extract of the present disclosure can provide a cosmetic composition with physiological and nutritional functionality due to its high quercetin content. A cosmetic composition containing the *Houttuynia cordata* fermentation extract according to the present disclosure can exhibit excellent skin barrier-strengthening, skin-moisturizing, antibacterial and regenerative effects.

## Description

### [Technical Field]

The present disclosure relates to a cosmetic composition containing a *Houttuynia cordata* fermentation extract fermented with lactic acid bacteria.

This application claims priority based on Korean Patent Application No. 10-2022-0179961, filed on December 21, 2022, the entire contents of which are incorporated herein by reference.

### [Background Art]

Fish mint is a perennial herb belonging to the family *Saururaceae*, and its scientific name is *Houttuynia cordata. Houttuynia cordata* is a medicinal herb called 'yakmomil' and was named 'fish mint' because its leaves have a fishy smell.

The entire leaves, stems and roots of *Houttuynia cordata* contain active ingredients, and physiologically active substances emitting characteristic odor are known to help with metabolism, purify the blood, promote kidney function, and relieve inflammation, excrete toxins from the body, etc. Therefore, it has been widely used as a folk medicine in China, Japan, etc. In traditional Chinese medicine, *Houttuynia cordata* is used for hypertension, constipation, diabetes, fever, swelling, detoxification, arthritis, inflammatory diseases, and skin diseases.

In this regard, Korean Patent Publication No. 10-2022-0091242 discloses a composition for improving skin wrinkles, which contains an extract of *Houttuynia cordata,* and Korean Patent Registration No. 10-2434747 discloses a cosmetic composition for an essential toner, which contains an extract of *Houttuynia cordata.*

However, since it is known that when the extract of *Houttuynia cordata* is used as is, the effect is significantly weakened compared to general chemical substances, there is a need for a fermentation method, etc. to enhance the physiological ingredients of the extract of *Houttuynia cordata* and their activity.

### [References of Related Art]

### [Patent Documents]

(Patent Document 1) Korean Patent Publication No. 10-2021-0091242 (2022.06.30).
(Patent document 2) Korean Patent Registration No. 10-2434747 (2022.08.17).

### [Disclosure]

### [Technical Problem]

The inventors of the present disclosure have conducted researches and made efforts to obtain a fermentation product of *Houttuynia cordata* with superior physiological and nutritional functionality. As a result, they have completed the present disclosure by discovering that a *Houttuynia cordata* fermentation extract fermented with specific lactic acid bacteria exhibits excellent skin barrier-strengthening, skin-moisturizing, antibacterial and regenerative effects.

Accordingly, the present disclosure is directed to providing a cosmetic composition containing a *Houttuynia cordata* fermentation extract fermented with lactic acid bacteria.

### [Technical Solution]

The present disclosure provides a cosmetic composition containing a *Houttuynia cordata* fermentation extract fermented with lactic acid bacteria.

The lactic acid bacteria may be one or more strain selected from a group consisting of the genus *Bacillus,* the genus *Streptococcus*, the genus *Lactococcus,* the genus *Enterococcus,* the genus *Lactobacillus,* the genus *Pediococcus,* the genus *Leuconostoc*, the genus *Weissella*, and the genus *Bifidobacterium*.

The lactic acid bacteria may be *Bacillus coagulans* KK7 (KCTC 19023P).

The *Houttuynia cordata* fermentation extract contains quercetin.

The cosmetic composition may be used for moisturizing skin or strengthening the skin barrier.

Meanwhile, the present disclosure provides a method for preparing a *Houttuynia cordata* fermentation extract, which includes a step of inoculating *Houttuynia cordata* with *Bacillus coagulans* KK7 (KCTC 19023P) and fermenting the same to obtain a *Houttuynia cordata* fermentation extract, and a *Houttuynia cordata* fermentation extract prepared thereby.

The fermentation may be performed at 20 to 60 °C for 1 to 5 days.

### [Advantageous Effects]

The *Houttuynia cordata* fermentation extract of the present disclosure can provide a cosmetic composition with physiological and nutritional functionality due to its high quercetin content.

A cosmetic composition containing the *Houttuynia cordata* fermentation extract according to the present disclosure can exhibit excellent skin barrier-strengthening, skin-moisturizing, antibacterial and regenerative effects.

### [Brief Description of Drawings]

FIG. 1 is a graph showing the result of measuring the amount of hyaluronic acid produced after treating with quercetin.
FIG. 2 is a graph showing the result of measuring the amount of hyaluronic acid produced after treating with the extracts of Example and Comparative Example.
FIG. 3 is a graph showing the result of measuring the amount of filaggrin produced after treating with quercetin.
FIG. 4 is a graph showing the result of measuring the amount of filaggrin produced after treating with the extracts of Example and Comparative Example.

### [Best Mode]

Hereinafter, the present disclosure is described in more detail. Terms or words used in this specification and claims should not be interpreted as limited to their usual or dictionary meanings, and should be interpreted as meanings and concepts that conform to the technical idea of the present disclosure based on the principle that an inventor can appropriately define the concept of terms in order to explain his or her own invention in the best way. Therefore, it should be understood that the configurations described in the exemplary embodiments described in this specification are only the most preferred embodiments of the present disclosure and do not represent all of the technical ideas of the present disclosure, and that there may be various equivalents and modifications that can replace them at the time of filing this application.

The cosmetic composition of the present disclosure contains a *Houttuynia cordata* fermentation extract fermented with lactic acid bacteria.

The *Houttuynia cordata* fermentation extract may be obtained by fermenting a *Houttuynia cordata* extract with lactic acid bacteria or by fermenting raw *Houttuynia cordata* with lactic acid bacteria.

The *Houttuynia cordata extract* may be one extracted using water or a C₁-C₄ alcohol as a solvent. But, specifically, it may be one extracted using hot water.

The solvent may be used in an amount 5 to 20 times, specifically 7 to 15 times, the weight of *Houttuynia cordata.* If the solvent is used in a smaller amount, the active ingredients may not be extracted sufficiently and the extraction yield may decrease. And, if it is used in a larger amount, there is a problem that the extraction process is inefficient.

The extraction may be performed using solvent extraction methods widely known in the art, such as immersion extraction, ultrasonic extraction, and vacuum extraction, although not being limited thereto.

When hot water is used during the extraction, the extraction may be performed at 40 to 100 °C for 0.5 to 24 hours, specifically at 60 to 90 °C for 1 to 12 hours. If the extraction is performed below the above-mentioned limited temperature range, the active ingredients may not be extracted sufficiently.

As the lactic acid bacteria, one or more strains selected from a group consisting of the genus *Bacillus,* the genus *Streptococcus*, the genus *Lactococcus,* the genus *Enterococcus*, the genus *Lactobacillus,* the genus *Pediococcus,* the genus *Leuconostoc*, the genus *Weissella*, and the genus *Bifidobacterium*, specifically the genus *Bacillus,* the genus *Lactobacillus,* the genus *Pediococcus,* or the genus *Lactococcus,* more specifically *Bacillus coagulans* KK7 (KCTC 19023P), may be used.

The *Houttuynia cordata* fermentation extract contains quercetin. The quercetin is a type of flavonoid and is found in fruits and vegetables. It is known to play a very important role in the prevention and treatment of various diseases as a powerful antioxidant, and various studies have shown that it provides skin nutrition, and has anti-allergic, anti-inflammatory and anti-cancer activities.

The *Houttuynia cordata* fermentation extract of the present disclosure has a higher quercetin content than general extracts, and may contain quercetin in an amount of about 60 to 180 mg/L. Therefore, the present disclosure can provide a cosmetic composition that is effective in moisturizing skin and strengthening the skin barrier by promoting the production of hyaluronic acid and filaggrin.

The cosmetic composition of the present disclosure may contain ingredients commonly used in cosmetic compositions, and may contain conventional auxiliary agents such as antioxidants, stabilizers, solubilizers, vitamins, pigments, and fragrances, as well as carriers.

The cosmetic composition of the present disclosure may be prepared into any formulation commonly prepared in the art. For example, it may be formulated into a solution, a suspension, an emulsion, a paste, a gel, a cream, a lotion, a powder, a soap, a surfactant-containing cleanser, an oil, a powder foundation, an emulsion foundation, a wax foundation, a spray, etc., although not being limited thereto.

More specifically, it may be prepared into the form of a softening lotion, a nourishing lotion, a nourishing cream, a massage cream, an essence, an eye cream, a cleansing cream, a cleansing foam, a cleansing water, a pack, a spray or a powder.

When the formulation of the present disclosure is a paste, cream or gel, animal oil, vegetable oil, wax, paraffin, starch, tragacanth, cellulose derivatives, polyethylene glycol, silicone, bentonite, silica, talc, zinc oxide, etc. may be used as a carrier component.

When the formulation of the present disclosure is a powder or spray, lactose, talc, silica, aluminum hydroxide, calcium silicate or polyamide powder may be used as a carrier component. Particularly, in the case of a spray, a propellant such as chlorofluorohydrocarbon, propane/butane or dimethyl ether may be included additionally.

When the formulation of the present disclosure is a solution or emulsion, a solvent, a solubilizer or an emulsifier is used as a carrier component. For example, water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, or fatty acid ester of sorbitan may be used.

When the formulation of the present disclosure is a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, tragacanth, etc. may be used as a carrier component.

When the formulation of the present disclosure is a surfactant-containing cleanser, an aliphatic alcohol sulfate, an aliphatic alcohol ether sulfate, a sulfosuccinic acid monoester, an isethionate, an imidazolinium derivative, methyl taurate, sarcosinate, a fatty acid amide ether sulfate, an alkyl amidobetaine, a fatty alcohol, a fatty acid glyceride, a fatty acid diethanolamide, a vegetable oil, a lanolin derivative, an ethoxylated glycerol fatty acid ester, etc. may be used as a carrier component.

When the cosmetic composition of the present disclosure is a soap, surfactant-containing cleanser or surfactant-free cleansing formulation, it may be wiped off, removed or washed off with water after being applied to the skin. As specific examples, the soap may be a liquid soap, a powder soap, a solid soap or an oil soap, the surfactant-containing cleansing formulation may be a cleansing foam, a cleansing water, a cleansing towel or a cleansing pack, and the surfactant-free cleansing formulation may be a cleansing cream, a cleansing lotion, a cleansing water or a cleansing gel, although not being limited thereto.

In addition, the *Houttuynia cordata* fermentation extract may be included in a pharmaceutical composition for external use on the skin, and the pharmaceutical composition may be in the form of a cream, a gel, a patch, a spray, an ointment, a paste, a lotion, a liniment, a paste or a cataplasm, although not being limited thereto.

Meanwhile, the present disclosure provides a method for preparing a *Houttuynia cordata* fermentation extract, which includes a step of inoculating *Houttuynia cordata* with *Bacillus coagulans* KK7 (KCTC 19023P) and fermenting the same to obtain a *Houttuynia cordata* fermentation extract.

The inoculation may be made using an extract of *Houttuynia cordata,* and the extract of *Houttuynia cordata* may be specifically a hot water extract, although not being limited thereto. The inoculation may also be made using raw *Houttuynia cordata.*

The *Bacillus coagulans* KK7 (KCTC 19023P) strain may be inoculated at a concentration of 1 to 10%, specifically 3 to 7%, relative to the weight of the *Houttuynia cordata* extract or raw *Houttuynia cordata.* If the concentration of the inoculated lactic acid bacteria is below 1%, fermentation may not occur sufficiently. And, if it exceeds 10%, over-fermentation may occur.

The fermentation may be performed at 20 °C to 60 °C and for 1 to 10 days. If the fermentation occurs below the ranges, fermentation may not occur sufficiently. And, if the fermentation occurs above the ranges, over-fermentation may occur.

The fermentation can be performed at 20 °C to 60 °C for 1 to 5 days, specifically at 30 to 40 °C for 3 to 4 days. If the fermentation occurs below the ranges, fermentation may not occur sufficiently. And, if the fermentation occurs above the ranges, over-fermentation may occur.

Hereinafter, the present disclosure will be described in detail through examples and test examples to specifically explain the present disclosure. However, the examples according to the present disclosure may be modified in various different forms, and the scope of the present disclosure should not be construed as being limited to the examples described below. The examples of the present disclosure are provided to more fully describe the present disclosure to those having ordinary skill in the art.

### [Example] Preparation of Houttuynia cordata fermentation extract using lactic acid bacteria

1 g of a kimchi sample was suspended in sterilized physiological saline and diluted to 10⁻⁶ to prepare a suspension. Then, 100 µL of the suspension was dispensed to a BDTM tryptic soy agar (pancreatic digest of casein 15 g·L⁻¹, papaic digest of soybean meal 5 g·L⁻¹, sodium chloride 5 g·L⁻¹, agar 5 g·L⁻¹, Product No. 236950) medium. An isolated microbial strain was obtained by subculturing a single colony in a medium for 48 hours under dark conditions at 30 ± 1 °C and pH 7.3 ± 0.2, and the strain was finally identified as *Bacillus coagulans* KK7 through 16S-rRNA sequencing. The *Bacillus coagulans* KK7 strain was deposited at the Korean Collection for Type Cultures of the Korea Research Institute of Bioscience and Biotechnology on July 27, 2022, and was assigned the accession number KCTC 19023P.

The isolated strain was subcultured in a tryptic soy agar medium with 24-hour intervals under dark conditions at 30 ± 1 °C and pH 7.3 ± 0.2.

Next, after adding 10 volume equivalents of purified water to dried *Houttuynia cordata,* an extract from which *Houttuynia cordata* was removed was obtained by performing hot water extraction at 80 °C for 60 minutes. After inoculating the cultured *Bacillus coagulans* KK7 strain to the extract at 5.0% of the extract weight and performing fermentation at 35 °C for 3 days, a *Houttuynia cordata* fermentation extract was obtained by removing the bacteria. After the filtered extract was concentrated under reduced pressure and the moisture was completely removed using a freeze dryer, an extract powder was obtained and used in the experiment.

### [Comparative Example 1] Preparation of Houttuynia cordata extract

After adding 10 volume equivalents of purified water to dried *Houttuynia cordata,* an extract from which the *Houttuynia cordata* was removed was obtained by performing hot water extraction at 80 °C for 60 minutes. Then, after concentrating the extract under reduced pressure and completely removing moisture using a freeze dryer, the obtained extract powder was used in the experiment.

### [Test Example 1] Analysis of quercetin content

The physiological ingredients of the extracts of the example and the comparative example were compared. The quercetin content of the extracts of the example and the comparative example was confirmed through HPLC analysis using a Shim-pack GIS C18 column. 100 mL of each of the extracts of the example and the comparative example was subjected to liquid-liquid extraction by adding 200 mL of ethyl acetate. After that, the ethyl acetate layer was recovered and concentrated under reduced pressure in a water bath at about 30 °C. The completely concentrated sample was dissolved in 10 mL of methanol and filtered with a syringe filter, and the quercetin content was measured. A standard was prepared by dissolving in methanol to a concentration of 0.5 to 200 µg/mL and filtering with a syringe filter. After the measurement, a peak area was calculated and a standard calibration curve was created using a regression equation. Distilled water containing 0.1% trifluoroacetic acid and acetonitrile were used as a mobile phase, and the flow rate was 1.0 mL/min. A PDA detector (371 nm) was used for detection. Measurement was made for 60 minutes in a gradient mode while changing the proportion of acetonitrile.

**[Table 1]**

| Sample | Quercetin content (mg/L) | S.D. |
|---|---|---|
| Example | 123.03 | 0.10 |
| Comparative Example | 31.69 | 0.04 |

As shown in Table 1, the *Houttuynia cordata* fermentation extract of the example showed a very high quercetin content, confirming that the content of quercetin increased due to the fermentation by lactic acid bacteria.

### [Test Example 2] Culturing of skin keratinocytes

HaCaT cells, which are skin keratinocytes, were provided by Cell Line Service GmbH (Germany). The cells were cultured in 5% CO₂ incubator at 37 °C using Dulbecco's modified Eagle's medium (DMEM) containing 1% penicillin-streptomycin and 10% fetal bovine serum (FBS). Subculturing was performed with 2-3 day intervals.

### [Test Example 3] Evaluation of cytotoxicity

### EZ-cytox assay

The EZ-cytox assay is a representative method for measuring cell viability that utilizes the principle that a water-soluble tetrazolium salt (WST) reacts with the dehydrogenase of living cells to produce an orange-colored, water-soluble formazan.

To examine cytotoxicity, HaCaT cells were seeded at 1.0x10⁴ cells/well in a 96-well plate using DMEM medium containing 10% FBS, and cultured under the condition of 37 °C and 5% CO₂ for 24 hours. After exchanging the medium with serum-free DMEM, the cultured cells were treated with a sample to be evaluated. After that, EZ-cytox was added to each well and reacted under the condition of 37 °C and 5% CO₂ for 30 minutes, and then absorbance was measured at 450 nm using a microplate reader. The average absorbance value for each sample group was calculated, and this value was compared with the absorbance value of the control group to evaluate cell viability.

**[Table 2]**

| | Cytotoxicity | |
|---|---|---|
| | Concentration | HaCaT cell growth rate (%) |
| Untreated group | - | 100.0 ± 1.2 |
| Example (µg/mL) | 250 | 98.5 ± 1.0 |
| | 500 | 97.7 ± 1.4 |
| | 1000 | 96.1 ± 1.7 |
| Comparative Example (µg/mL) | 250 | 101.4 ± 1.8 |
| | 500 | 108.4 ± 1.9 |
| | 1000 | 111.2 ± 2.3 |
| Quercetin (µM) | 25 | 104.3 ± 1.3 |
| | 50 | 105.9 ± 1.0 |
| | 100 | 109.4 ± 2.1 |
| | 200 | 92.8 ± 2.8 |

As shown in Table 2, none of the example, comparative example or quercetin exhibited cytotoxicity for the skin keratinocytes.

### [Test Example 4] Effect of increased production of moisturizing factor hyaluronic acid (HA)

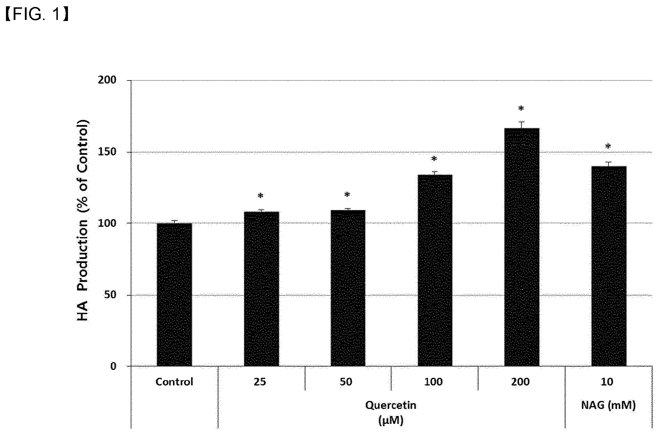

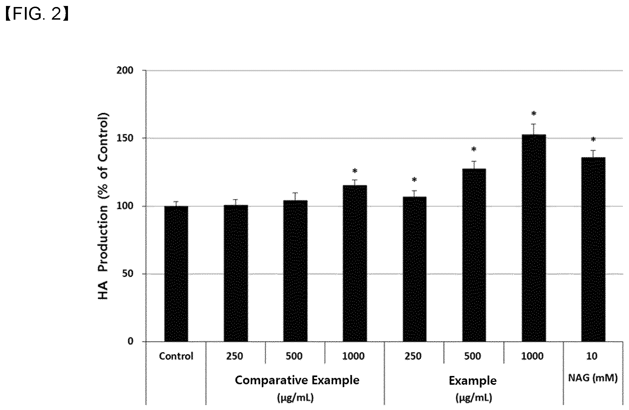

HaCaT cells were seeded at 1.0x10⁵ cells/well in a 24-well plate and cultured for 24 hours under the condition of 37 °C and 5% CO₂. After the cultured cell medium was replaced with serum-free DMEM, a sample was treated at different concentrations and the cells were cultured for 24 hours. Afterwards, the supernatant from each test group was collected and the amount of hyaluronic acid (HA) produced in the medium was measured. A control group was treated with N-acetylglucosamine at a concentration of 10 mM. The measurement of hyaluronic acid production was performed using the Hyaluronan Quantikine ELISA kit (R&D Systems) according to the method provided by the manufacturer. The result of measuring the amount of hyaluronic acid produced depending on the quercetin treatment is shown in FIG. 1, and the result of measuring the amount of hyaluronic acid produced depending on the treatment with the extracts of the example and comparative example is shown in FIG. 2.

As shown in FIG. 1, quercetin promoted the production of the moisturizing factor hyaluronic acid. In particular, as shown in FIG. 2, it was confirmed that the fermentation extract of the example promoted the production of the moisturizing factor hyaluronic acid more than the extract of the comparative example.

### [Test Example 5] Effect of increased production of moisturizing factor filaggrin (FLG)

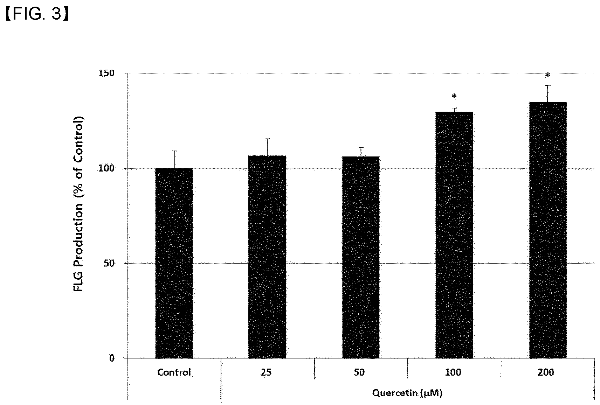

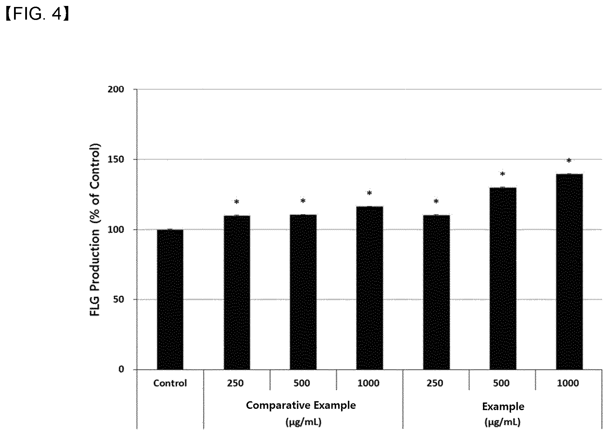

HaCaT cells were seeded at 1.0x10⁵ cells/well in a 24-well plate and cultured for 24 hours under the condition of 37 °C and 5% CO₂. After the cultured cell medium was replaced with serum-free DMEM, a sample was treated at different concentrations and the cells were cultured for 24 hours. Afterwards, the cells of each test group were lysed and proteins were collected. Then, the amount of filaggrin produced in the culture medium extract was measured. The measurement of filaggrin production was performed using the Filaggrin ELISA kit (Cusabio Biotechnology Co., Ltd) according to the method provided by the manufacturer. The result of measuring the amount of filaggrin produced depending on the quercetin treatment is shown in FIG. 3, and the result of measuring the amount of filaggrin produced depending on the treatment with the extracts of the example and comparative example is shown in FIG. 4.

As shown in FIG. 3, quercetin promoted the production of the moisturizing factor filaggrin. And, as shown in FIG. 4, it was confirmed that the fermentation extract of the example further promoted the production of the moisturizing factor filaggrin as compared to the extract of the comparative example.

## Claims

1. A cosmetic composition comprising a *Houttuynia cordata* fermentation extract fermented with lactic acid bacteria.

2. The cosmetic composition according to claim 1, wherein the lactic acid bacteria are one or more strain selected from a group consisting of the genus *Bacillus,* the genus *Streptococcus,* the genus *Lactococcus,* the genus *Enterococcus,* the genus *Lactobacillus,* the genus *Pediococcus,* the genus *Leuconostoc,* the genus *Weissella,* and the genus *Bifidobacterium.*

3. The cosmetic composition according to claim 2, wherein the lactic acid bacteria are *Bacillus coagulans* KK7 (KCTC 19023P).

4. The cosmetic composition according to claim 1, wherein the *Houttuynia cordata* fermentation extract comprises quercetin.

5. The cosmetic composition according to claim 1, wherein the composition has superior skin-moisturizing and skin barrier-strengthening effects.

6. A method for preparing a *Houttuynia cordata* fermentation extract, comprising a step of obtaining a *Houttuynia cordata* fermentation extract by inoculating *Houttuynia cordata* with *Bacillus coagulans* KK7 (KCTC 19023P) and fermenting the same.

7. The method for preparing a *Houttuynia cordata* fermentation extract according to claim 6, wherein the fermentation is performed at 20 to 60 °C for 1 to 5 days.

8. *A Houttuynia cordata* fermentation extract prepared by the preparation method according to claim 6.
